# EUROPÄISCHE PATENTSCHRIFT

(11) **EP 0 352 596 B1**
(45) Veröffentlichungstag und Bekanntmachung des Hinweises auf die Patenterteilung: **08.12.1993**
(21) Anmeldenummer: 89113093.2
(22) Anmeldetag: 17.07.1989
(51) Int. Cl.: A61B 5/04

(54) **Saugelektrodenvorrichtung für elektromedizinische Geräte**
Suction electrodes for electro-medical apparatus
Jeu d'électrodes en forme de ventouse relié à un appareil électro-médical

(30) Priorität: 29.07.1988 DE 8809707 U
(43) Veröffentlichungstag der Anmeldung: 31.01.1990
(73) Patentinhaber: SIEMENS AKTIENGESELLSCHAFT, 80333 München (DE)
(72) Erfinder: Sieber, Gotthold, Dipl.-Ing. (FH), D-8520 Erlangen (DE)

(56) Entgegenhaltungen:
- EP-A- 0 199 694
- DE-A- 2 742 058
- DE-B- 1 939 523
- DE-B- 2 513 445
- DE-C- 3 502 913
- DE-U- 1 952 893

## Beschreibung

Die Erfindung betrifft eine Saugelektrodenvorrichtung zur Therapie oder Diagnostik, mit einer durch Unterdruck zu applizierenden Saugelektrode, die einen Saugkopf, der durch eine Leitung an ein den zu applizierenden Strom lieferndes elektromedizinisches Gerät sowie an einen Druckerzeuger anschließbar ist und eine Strahldüse trägt, aufweist, mit einer am Saugkopf befestigbaren Saugglocke und mit elektrischen Anschlüssen für wenigstens zwei daran und in der Saugglocke lösbar befestigten Elektroden.

Gemäß dem Stand der Technik kann der Druckerzeuger aus einem hinter oder vor der Saugdüse angeschlossenen Druck- bzw. Sauggebläse bestehen, wobei die Strahldüse über eine Ansaugöffnung einen Unterdruck in der Saugglocke erzeugt. Nach weiterer bekannter Ausführung kann die Saugglocke auch direkt an eine Unterdruckleitung angeschlossen werden.

Bei einer bekannten Saugelektrodenvorrichtung dieser Art (Sonderdruck aus ORTHOPÄDISCHE PRAXIS 11/83, Seite 785-796, Medizinisch Literarische Verlagsgesellschaft mbH, 3110 Uelzen; "Schmerztherapie mit nieder- und hochfrequenten Strömen" von H. Thom), die zur Reizstromtherapie dient, wird dem Patienten der zu applizierende Strom eines Reizstromgerätes über Saugelektroden zugeführt. Hierzu wird eine dreipolige Saugelektrode mit speziellen Elektroden verwendet, die sich problemlos am Patienten anlegen lassen. Dabei besteht die Saugelektrode aus einem Saugkopf aus Kunststoff, an den eine elektrische und eine Druckluftstrom-Leitung angeschlossen sind. Mittels einer im Saugkopf angeordneten Strahldüse entsteht durch den Druckluftstrom im Innenraum einer auf den Saugkopf aufsetzbaren elastischen Saugglocke ein Unterdruck, der den notwendigen Haftdruck für die Applikation der Elektroden am Patienten erzeugt, wobei die Strahldüse nach dem Injektor-Verfahren ähnlich einer Wasserstrahlpumpe arbeitet. In den Saugkopf der bekannten Saugelektrode sind ferner drei mit der elektrischen Zuleitung kontaktierte Steckerbuchsen eingegossen. Die aus Metallscheiben bestehenden Elektroden sind in napfförmige Aufnahmen eines elastischen Einsatzes eingeklebt oder eingeschweißt, wobei der trichterförmige, aus Silikongummi bestehende Einsatz im Bereich seiner bodenseitigen Trichteröffnung einen Stecker trägt, der mit seinen drei Steckerstiften in die Steckerbuchsen des Saugkopfes einsetzbar ist und in dessen elastischer Trichterwand die elektrischen Zuleitungen zu den Elektroden eingebettet sind. Zur Verbesserung des Kontaktes zwischen den Elektroden und der Hautoberfläche des Patienten an der Applikationsstelle dienen als Elektrodenzwischenlage auswechselbare Kontaktfilze, ein Vlies od.dgl. und es wird eine Elektrodenkontaktflüssigkeit verwendet. Zum Säubern der Saugelektrode muß der die Elektroden tragende Einsatz vom Saugkopf gelöst und aus der Saugglocke genommen werden. Durch häufigeres Auswechseln der Einsätze und die damit unumgängliche Verwindung derselben kann es zum Bruch der in der trichterförmigen Wand der Einsätze verlegten Leitungsdrähte kommen. Auch kann der durch die bodenseitige Trichteröffnung und eine Bohrung im Saugkopf zur Strahldüse geführte Ansaugkanal im Falle einer Verschmutzung nur relativ schwierig gereinigt werden, da er nicht frei zugänglich ist. Der elastische, die Elektroden aufnehmende Einsatz erfordert zudem einen beachtlichen Fertigungsaufwand.

Aus der DE-AS 19 39 523 ist eine Saugelektrodenvorrichtung für elektromedizinische Geräte zur Therapie oder Diagnostik mit mindestens einer durch Unterdruck unter Verwendung einer elektrisch leitenden Kontaktflüssigkeit zu applizierenden Saugelektrode bekannt, die eine an eine Saugpumpe angeschlossene Saugglocke aufweist. Diese einpolige Saugelektrode besitzt eine Elektrode, wobei der Ansaugkanal dabei durch die Elektrodenplatte hindurch bis ins Innere des Rohrkanals der Strahldüse nahe der engen Rohrstelle gebohrt ist. Das die Unterdruckdüse bildende Metallrohr sowie die fest damit verbundene Elektrodenplatte sitzen in einem Elektrodengehäuse, das applikationsseitig eine umlaufende Dichtlippe aufweist. Wird die Saugelektrode mit der Dichtlippe auf der Hautoberfläche eines Patienten aufgesetzt und wird dann anschließend vom einen Ende der Unterdruckdüse durch eine Gaspumpe beispielsweise elektrisch isolierendes Preßgas durch die Unterdruckdüse geblasen, so entsteht an der engen Stelle ein Unterdruck, der sich über den Ansaugkanal zur Applikationsstelle fortsetzt. Die Saugelektrode saugt sich daraufhin auf der Hautoberfläche an. Elektrodenkontaktflüssigkeit, mit der z.B. zur Herstellung besseren Kontaktes ein zwischen Elektrodenplatte und Hautoberfläche des Patienten an der Applikationsstelle eingesetzter Elektrodenschwamm getränkt ist und die während der Behandlungszeit wegen des Unterdruckes über den Ansaugkanal in den Rohrkanal angesaugt wird, wird über das frei mündende Ende des Rohrkanals in die Atmosphäre versprüht. Die Elektrodenkontaktflüssigkeit kann also nicht in die Gaspumpe gelangen und diese verschmutzen. Bei der bekannten Saugelektrode ist die elektrisch leitende Elektrode mit der Unterdruckdüse fest verbunden. Außerdem sind die elektrisch leitende Elektrode und die Unterdruckdüse im Elektrodengehäuse fest eingebaut. In Fällen, bei denen unterschiedlich große, elektrisch leitende Elektroden eingesetzt werden sollen, müssen also für jede Elektrodengröße komplette Sätze mit verschiedenen Saugelektroden bereitgestellt werden. Auch bei dieser bekannten, einpoligen Saugelektrode läßt sich der Ansaugkanal im Falle einer Verschmutzung nur schwierig reinigen, da er durch die Elektrode hindurch nur schwer zugänglich ist und nicht durchstoßen werden kann.

Eine aus der CH-PS 301 242 bekannte elektromedizinische, einpolige Saugelektrode ist mittels einer Saugleitung an einen Unterdruckerzeuger anzuschließen und gekennzeichnet durch einen Isolierkörper, der nach einer Seite hin zu einem Saugnapf ausgebildet und mit einer senkrecht zur Achse des Saugnapfes eingeführten Metallbuchse für den Anschluß der Saugleitung und der elektrischen Zuleitung versehen ist. Dabei ist die Metallbuchse durch eine Öffnung mit dem Saugnapf verbunden, indem zentral eine sich im Saugnapf tellerförmig ausbreitende Elektrode angeordnet ist. Die eigentliche Elektrode, die sich im Saugnapf tellerförmig ausbreitet, ist mit einem Bolzen versehen, mit dem sie in die Metallbuchse eingeschraubt ist, so daß die Elektrode in Richtung des Bolzens verstellbar ist.

Schließlich ist aus der EP-A-01 99 694 ein mittels Unterdruck befestigbarer Halter mit Elektroanschluß bekannt, in dessen Gehäuse ein elastisches Zwischenstück einsetzbar ist, in welches dann eine EKG-Elektrode eingeknüpft werden kann. Dabei handelt es sich wiederum um eine einpolige Saugelektrode mit einer einzigen, zentral und starr angeordneten Elektrode.

Aufgabe vorliegender Erfindung ist es, eine mehrpolige Saugelektrode der eingangs genannten Art so auszubilden, daß ein einfaches Reinigen des Saugkopfes sowie Wechseln der Elektroden möglich und dabei eine Bruchgefahr der elektrischen Elektrodenzuleitungen ausgeschlossen ist.

Diese Aufgabe wird erfindungsgemäß durch die kennzeichnen den Merkmale des Anspruches 1 gelöst. Gemäß der Erfindung sind die Elektroden der mehrpoligen, insbesondere zwei- oder dreipoligen, Saugelektrode einfach von den feststehenden Anschlüssen des Saugkopfes abnehmbar und wieder aufsteckbar bzw. durch Elektroden anderer Größe oder Form austauschbar. Die bewegliche Anordnung der Elektroden auf den Anschlüssen erübrigt einen zusätzlichen elastischen Träger für die Elektroden und ermöglicht dennoch ein problemloses Anlegen an die Haut des Patienten.

Weitere Vorteile und Einzelheiten der Erfindung ergeben sich aus der nachfolgenden Beschreibung eines Ausführungsbeispiels anhand der Zeichnung und in Verbindung mit den Ansprüchen 2 bis 8.

Es zeigen:
Figur 1 eine dreipolige Saugelektrode gemäß der Erfindung in perspektivischer Darstellung, wobei die Saugglocke von dem die Elektroden tragenden Saugkopf abgenommen ist,
Figur 2 eine Saugelektrode nach Figur 1 im Schnitt,
Figur 3 eine Art der Befestigung einer elektrisch leitenden Elektrode der Saugelektrode an einem feststehenden Anschlußstift des Saugkopfes, wobei der Anschlußstift einen kugelförmigen Kopf aufweist, auf den eine Halteklammer der Elektrode zu deren schwenkbarer Befestigung aufsetzbar ist,
Figur 4 eine Draufsicht auf die Rückseite des Saugkopfes, aus der bei abgenommener Gehäusekappe der Raum zum Anschluß der elektrischen Zuleitung und des Zuführungsschlauches erkennbar ist.

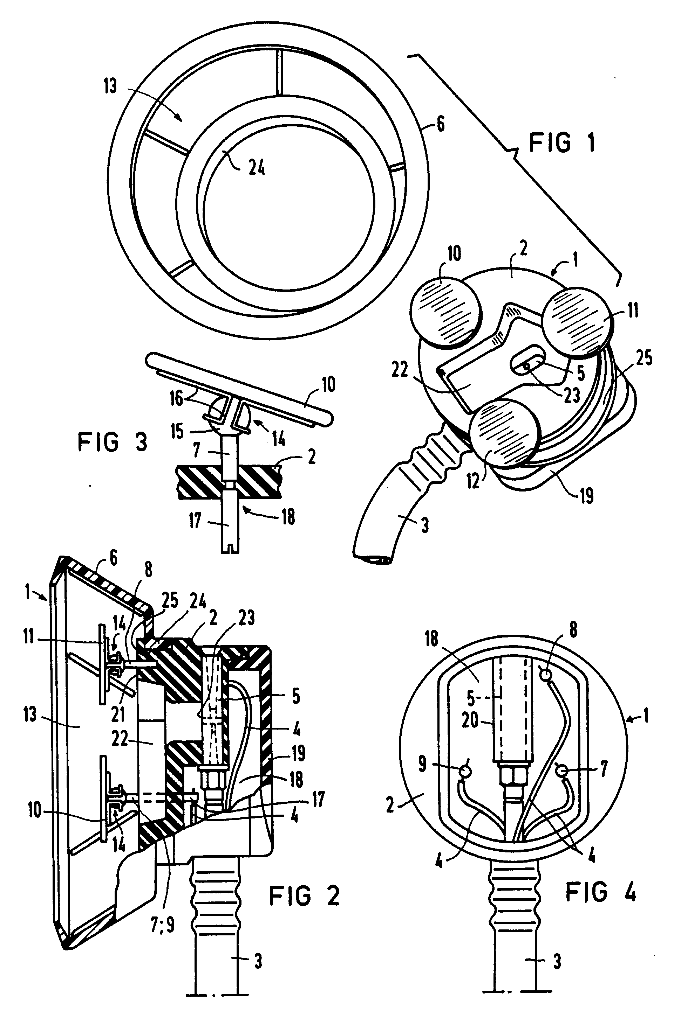

Bei dem Ausführungsbeispiel nach der Figur 1 umfaßt die Saugelektrodenvorrichtung eine dreipolige Saugelektrode 1, die über einen Zuführungsschlauch 3 an einen Drucklufterzeuger und eine elektrische Zuleitung 4 an ein den zu applizierenden Strom lieferndes, nicht dargestelltes elektromedizinisches Gerät, z.B. Reizstromgerät, angeschlossen ist. Die Saugelektrode 1 besteht aus einem Saugkopf 2 aus Kunststoff und einer Saugglocke 6, wobei der Zuführungsschlauch 3 an eine Strahldüse 5 im Saugkopf angeschlossen und die elektrische Zuleitung 4 zu elektrischen Anschlüssen 7, 8, 9 des Saugkopfes geführt ist. Ferner weist die Saugelektrode 1 drei z.B. knopfförmig ausgebildete, elektrisch leitende Elektroden 10, 11, 12 auf, die beweglich auf den elektrischen Anschlüssen 7, 8, 9 sitzen, jedoch zum Reinigen des Saugkopfes bzw. der Saugglocke oder zum Auswechseln durch Elektroden anderer Größe oder Form von den Anschlüssen 7, 8, 9 leicht abnehmbar sind. In Figur 1 sind als Verbindung zwischen den elektrischen Anschlüssen und den Elektroden männliche bzw. weibliche Steckelemente 14 vorgesehen.

Bei der in Figur 3 als Einzelheit gezeichneten Ausführung ist ein elektrischer Anschluß in Form eines Anschlußstiftes 7 dargestellt, der in einem kugelförmigen Kopf 15 endet und auf den eine elektrisch leitende und an der Elektrodenunterseite befestigte, federnde Halteklammer 16 aufsetzbar ist. Wie die Figur 1 zeigt, ragen die in den Kunststoff-Saugkopf 2 eingegossenen oder eingespritzten Anschlußstifte 7, 8, 9 in den hohlen Innenraum 13 der Saugglocke 6 und können gut zugängig mit den Elektroden 10, 11, 12 bestückt werden.

Auf der der Saugglocke abgewandten Rückseite des Saugkopfes 2 ist nach dem dargestellten Ausführungsbeispiel eine Gehäusekappe 19 vorgesehen, die einen Raum 18 im Saugkopf abdeckt. Im Raum 18 befindet sich eine Aufnahme 20 für die Strahldüse 5. Ferner sind im Raum 18 die elektrischen Anschlüsse von der elektrischen Zuleitung 4 zu den Anschlußstiften 7, 8, 9 vorgesehen, die dazu mit ihren unteren Enden 17 in den Raum 18 ragen und leicht zugänglich mit der Zuleitung 4 verbindbar sind.

Die aus elastischem Kunststoff (z.B. Silikongummi) bestehende Saugglocke 6 ist über einen eingezogenen Flanschring 24 in eine am Umfang des Saugkopfes 2 eingelassene Ringnut 25 einknüpfbar, wobei dann der Saugkopf den Boden der Saugglocke bildet. Auf der dem Innenraum 13 der Saugglocke zugewandten Seite weist der Saugkopf 2 eine Vertiefung 22 auf, die sich bis zur Ansaugöffnung 23 der Strahldüse erstreckt. In versetzter Anordnung sind an dem die Vertiefung 22 umgebenden Bodenrand 21 des Saugkopfes die Anschlußstifte 7, 8, 9 für die Elektroden 10, 11, 12 angeordnet, wobei die Ansaugöffnung 23 zur Reinigung gut zugängig ist.

## Patentansprüche

1. Saugelektrodenvorrichtung zur Therapie oder Diagnostik, mit einer durch Unterdruck zu applizierenden Saugelektrode (1), die einen Saugkopf (2) aufweist, der durch eine Leitung (3, 4) an ein den zu applizierenden Strom lieferndes elektromedizinisches Gerät so wie an einen Druckerzeuger anschließbar ist und eine Strahldüse (5) trägt, mit einer am Saugkopf (2) befestigbaren Saugglocke (6) und mit elektrischen Anschlüssen (7 - 9) für wenigstens zwei daran und in der Saugglocke lösbar befestigten Elektroden (10 - 12), **dadurch gekennzeichnet,** daß die in den Saugkopf (2) eingeführte elektrische Zuleitung (4) für die Elektroden (10, 11, 12) in aus dem Saugkopf in den hohlen Innenraum (13) der Saugglocke (6) ragenden, feststehenden Anschlüssen (7, 8, 9) endet und daß die Elektroden durch männliche oder weibliche Steckelemente (14) mit den Anschlüssen beweglich verbindbar sind.

2. Saugelektrodenvorrichtung nach Anspruch 1, **dadurch gekennzeichnet,** daß die in den Innenraum (13) der Saugglocke (6) ragenden Anschlüsse (7, 8, 9) kugelförmige Köpfe aufweisen.

3. Saugelektrodenvorrichtung nach Anspruch 1 und 2, **dadurch gekennzeichnet,** daß die Elektroden (10, 11, 12) auf ihrer der Applikationsseite abgewandten Unterseite elektrisch leitende, auf die Anschlüsse (7, 8, 9) aufsetzbare Halteklammern (16), Spangen, Hülsen mit Rasten oder Federzungen aufweisen.

4. Saugelektrodenvorrichtung nach Anspruch 1 bis 3, **dadurch gekennzeichnet,** daß der Saugkopf (2) mit jeweils zwei oder drei Elektroden (10, 11, 12) bestückbar ist und daß die Elektroden nach Form und Größe auswechselbar sind.

5. Saugelektrodenvorrichtung nach Anspruch 1, **dadurch gekennzeichnet,** daß die Anschlüsse (7, 8, 9) als Stifte ausgebildet und in den aus Kunststoff bestehenden Saugkopf (2) eingeformt sind, wobei die stiftförmigen Anschlüsse mit ihren den Elektroden (10, 11, 12) abgewandten unteren Enden (17) in einen ausgeformten Raum (18) des Saugkopfes ragen und an die elektrische Zuleitung (4) angeschlossen sind und daß dieser Anschlußraum (18) des Saugkopfes (2) durch eine Gehäusekappe (19) verschließbar ist.

6. Saugelektrodenvorrichtung nach Anspruch 5, **dadurch gekennzeichnet,** daß in den Raum (18) des Saugkopfes (2) eine Aufnahme (20) für die Strahldüse (5) eingeformt und der Anschluß der Strahldüse an einen Zuführungsschlauch (3) in diesem Raum vorgesehen ist.

7. Saugelektrodenvorrichtung nach Anspruch 1, **dadurch gekennzeichnet,** daß der Saugkopf (2) an der den Boden der Saugglocke (6) verschließenden Seite eine zentral ausgesparte Vertiefung (22) mit Zugang zur Ansaugöffnung (23) der Strahldüse (5) aufweist und daß die Anschlußstifte (7, 8, 9) der Elektroden (10, 11, 12) außerhalb der Vertiefung am Bodenrand (21) des Saugkopfes angeordnet sind.

8. Saugelektrodenvorrichtung nach Anspruch 1, **dadurch gekennzeichnet,** daß die Saugglocke (6) aus elastischem Material besteht und mit einem bodenseitig eingezogenen Flanschring (24) in eine äußere Ringnut (25) des Saugkopfes (2) einknöpfbar ist.

## Claims

1. Suction electrode device for therapy or diagnosis, having a suction electrode (1) for application by means of low pressure, which suction electrode has a suction head (2) which can be connected by a line (3, 4) to an electro-medical apparatus supplying the current for application and to a pressure generator and carries a jet nozzle (5), having a suction bell (6) which can be secured to the suction head (2) and having electrical terminals (7 - 9) for at least two electrodes (10 - 12) which can be detachably secured thereto and in the suction bell, characterised in that the electrical supply lead (4) inserted in the suction head (2) for the electrodes (10, 11, 12) ends in fixed terminals (7, 8, 9) projecting from the suction head into the hollow interior space (13) of the suction bell (6), and in that the electrodes can be connected to the terminals in a movable manner by means of male or female plug-in elements (14).

2. Suction electrode device according to claim 1, characterised in that the terminals (7, 8, 9) projecting into the interior space (13) of the suction bell (6) have spherical heads.

3. Suction electrode device according to claims 1 and 2, characterised in that the electrodes (10, 11, 12) have, on their underside facing away from the application side, electrically conductive clamp carriers (16), clasps, sleeves with notches or spring tongues which can be placed on the terminals (7, 8, 9).

4. Suction electrode device according to claims 1 to 3, characterised in that the suction head (2) can be fitted with two or three respective electrodes (10, 11, 12) and in that the electrodes can be exchanged according to shape and size.

5. Suction electrode device according to claim 1, characterised in that the terminals (7, 8, 9) are formed as pins and are integrally formed in the suction head (2) consisting of plastics material, whereby the pin-type terminals project at their lower ends (17) facing away from the electrodes (10, 11, 12) into a space (18) formed in the suction head and are connected to the electrical supply lead (4), and in that this connection space (18) in the suction head (2) can be closed off by means of a housing cap (19).

6. Suction electrode device according to claim 5, characterised in that a seating (20) for the jet nozzle (5) is integrally formed in the space (18) in the suction head (2) and the connection of the jet nozzle to a supply tube (3) is provided in this space.

7. Suction electrode device according to claim 1, characterised in that the suction head (2) has on the side closing off the base of the suction bell (6) a centrally recessed cavity (22) with access to the intake opening (23) in the jet nozzle (5), and in that the terminal pins (7, 8, 9) of the electrodes (10, 11, 12) are arranged outside the cavity on the base edge (21) of the suction head.

8. Suction electrode device according to claim 1, characterised in that the suction bell (6) consists of elastic material and can be pushed into an outer annular groove (25) in the suction head (2) by means of a flange ring (24) inserted on the base side.

## Revendications

1. Dispositif à électrodes à succion pour la thérapie ou le diagnostic, comportant une électrode à succion (1) devant être appliquée sous l'effet d'une dépression, qui possède une tête de succion (2) qui peut être raccordée par une canalisation (3,4) à un appareil électromédical délivrant le courant à appliquer, ainsi qu'un générateur de pression, et porte une buse de projection (5), et comportant une ventouse (6) pouvant être fixée sur la tête de succion (2), et des bornes électriques (7-9) pour au moins deux électrodes (10-12) fixées de façon amovible à ces bornes et dans la ventouse, caractérisé par le fait que le conducteur d'alimentation électrique (4), introduit dans la tête de succion (2), pour les électrodes (10,11,12) se termine par des bornes fixes (7,8,9), qui font saillie hors de la tête de succion et pénètrent dans la cavité intérieure (13) de la ventouse (6), et que les électrodes peuvent être raccordées, avec possibilité de déplacement, aux bornes au moyen d'éléments à enfichage mâles et femelles (14).

2. Dispositif à électrodes à succion suivant la revendication 1, caractérisé par le fait que les bornes (7,8,9), qui pénètrent dans l'espace intérieur (13) de la ventouse (6), possèdent des têtes de forme sphérique.

3. Dispositif à électrodes à succion suivant les revendications 1 et 2, caractérisé par le fait que les électrodes (10,11,12) possèdent, sur leur face inférieure tournée à l'opposé de la face d'application, des pinces de retenue (16), des mâchoires, des douilles comportant des éléments d'encliquetage ou des languettes à ressorts, qui sont électriquement conductrices et qui peuvent être emmanchées sur les bornes (7,8,9).

4. Dispositif à électrodes à succion suivant les revendications 1 à 3, caractérisé par le fait que la tête de succion (2) peut être pourvue de deux ou de trois électrodes (10,11,12) et que la forme et les dimensions des électrodes peuvent être modifiées.

5. Dispositif à électrodes à succion suivant la revendication 1, caractérisé par le fait que les bornes (7,8,9) sont réalisées sous la forme de broches et sont formées dans la tête de succion (2) réalisée en matière plastique, les bornes en forme de broches pénétrant, par leurs extrémités inférieures (17) tournées à l'opposé des électrodes (10,11,12), dans un espace (18), formé par moulage, de la tête de succion et étant raccordées au conducteur d'alimentation électrique (4), et que cet espace de raccordement (18) de la tête de succion (2) peut être fermée par un capuchon (19) d'un boîtier.

6. Dispositif à électrodes à succion suivant la revendication 5, caractérisé par le fait qu'un logement (20) pour la buse de projection (5) est formé par moulage dans l'espace (18) de la tête de succion (2) et que le raccordement de la buse de projection à un tuyau d'alimentation (3) est prévu dans cet espace.

7. Dispositif à électrodes à succion suivant la revendication 1, caractérisé par le fait que la tête de succion (2) possède, sur le côté, qui ferme le fond de la ventouse (6), un renfoncement central (22) qui réalise l'accès à l'ouverture d'aspiration (23) de la buse de projection (5) et que les broches de raccordement (7,8,9) des électrodes (10,11,12) sont disposées en dehors du renfoncement ménagé dans le bord (22) du fond de la tête de succion.

8. Dispositif à électrodes à succion suivant la revendication 1, caractérisé par le fait que la ventouse (6) est réalisée en un matériau élastique et peut être encliquetée, par une bride annulaire (24), qui est rentrée côté fond, dans une gorge annulaire extérieure (25) de la tête de succion (2).
